# EUROPÄISCHE PATENTSCHRIFT

(11) **EP 3 034 619 B1**
(45) Veröffentlichungstag und Bekanntmachung des Hinweises auf die Patenterteilung: **17.10.2018**
(21) Anmeldenummer: 14198311.4
(22) Anmeldetag: 16.12.2014
(51) Int. Cl.: C12Q 1/6806

(54) **Modifizierte Oligonukleotide und Verfahren zum Einschleusen von Primern oder Kontrollmolekülen oder Sonden in geschlossene PCR-Systeme**
Modified oligonucleotides and method for feeding primers or control molecules or probes in closed PCR systems
Oligonucléotide modifié et procédé d'éclusage de primaires ou de molécules de commande ou de sondes dans des systèmes PCR fermés

(43) Veröffentlichungstag der Anmeldung: 22.06.2016
(73) Patentinhaber: microBIOMix GmbH, 93053 Regensburg (DE)
(72) Erfinder: REISCHL, Udo, 93107 Thalmassing (DE); GESSNER, André, 93059 Regensburg (DE); HIERGEIST, Andreas, 93051 Regensburg (DE)
(74) Vertreter: Hannke, Christian

(56) Entgegenhaltungen:
- WO-A1-96/37630
- DE-A1-102011 055 247
- ALDRED ANGELA R ET AL: "Synthesis of rat transferrin in Escherichia coli containing a recombinant bacteriophage", BIOCHEMICAL AND BIOPHYSICAL RESEARCH COMMUNICATIONS, vol. 122, no. 3, 1984, - 1984, pages 960-965, XP028831191, ISSN: 0006-291X, DOI: 10.1016/0006-291X(84)91185-9

## Beschreibung

Die vorliegende Erfindung betrifft eine Verwendung für ein modifiziertes Oligonukleotid, welches insbesondere eine Sonde und/oder einen Primer für PCR-Reaktionen umfasst.

Weiterhin betrifft die Erfindung ein Verfahren zum Einschleusen eines Primers und/oder einer Sonde in ein geschlossenes PCR-System.

Außerdem ist ein Kit Gegenstand der Erfindung. Ein solches Kit umfasst ein modifiziertes Oligonukleotid sowie ein Auxiliar, zur Freisetzung eines PCR-Primers und/oder einer Sonde aus diesem Oligonukleotid.

Weiterhin offenbart ist ein Kit, welches ein modifiziertes Oligonukleotid sowie ein PCR-System mit einer für das modifizierte Oligonukleotid kompatiblen Schleuse umfasst.

Die Polymerase-Kettenreaktion (Polymerase Chain Reaction, im Folgenden als PCR bezeichnet) hat sich zu einem wichtigen Instrument in einer Vielzahl von Prozessen entwickelt. Sie wird in vielen Labors routinemäßig eingesetzt. Seit ihrer Entwicklung wurden vielfältige Veränderungen und Weiterentwicklungen vorgenommen. Diese zielen insbesondere auf einen schnellen Temperaturwechsel und auf eine Miniaturisierung und somit auch eine Reduzierung des zu temperierenden Probenvolumens ab. Nachdem die chemischen und biophysikalischen Probleme einer schnellen und miniaturisierten Abarbeitung von diagnostischen PCR-Reaktionen in funktionierende Testsysteme umgesetzt wurden, basieren moderne Geräteplattformen zunehmend auf teil- bzw. vollautomatisierten und physikalisch geschlossenen Testkonzepten.

Die PCR wird vielfältig genutzt. Beispielsweise ist aus DE 10 2011 055 247 A1 eine Oligonukleotid-Sonde bekannt, die zwei Regionen, nämlich eine Sondenregion und eine Mediatorregion, aufweist. Beide Bereiche sind über eine Spaltstelle verbunden. Kann nach der Spaltung die Mediatorregion an eine auf einer Festphase gebundene und als Hairpin vorliegenden DNA binden, kann eine PCR erfolgen, welche in einer Auflösung der Hairpin-Struktur resultiert und ein Fluoreszenzsignal auslöst.

Auch in WO 96/37 630 A1 ist ein Verfahren beschrieben, welches PCR nutzt. Ein an einer Festphase gebundener Primer wird durch PCR verlängert. Nach der PCR wird der Primer jedoch gespalten, so dass das Primer-Verlängerungsprodukt nur wenige Nukleinsäuren des ursprünglichen Primers enthält. Darüber hinaus wird durch diese Spaltung das Primer-Verlängerungsprodukt von der Festphase gelöst.

DNA mit Schnittstellen, die durch enzymatische Spaltung an diesen Schnittstellen in kleinere Fragmente geteilt werden kann, ist aus Aldred, A. R. et al. "Systhesis of rat transferrin in Escherichia coli containing a recombinant bacteriophage", Biochemical and Biophysical Research Communications, 122(3) 1984, 960-965 bekannt.

Zunehmend werden zur PCR Systeme im Kartuschen- oder Chip-format eingesetzt. Derartige Systeme haben insbesondere den Vorteil, dass sie stark automatisiert betrieben werden und Fehler durch Bedienpersonal minimiert werden können. Daher sind geschlossene Systeme wesentlich weniger anfällig für Kontaminationsereignisse (falsch-positive Ergebnisse) sowie möglichen Bearbeitungsfehlern in den drei Einzelschritten: (i) der Nukleinsäureisolierung aus dem Probenmaterial, (ii) dem Ansetzen bzw. Komplettieren der jeweils spezifischen PCR-Reaktionskomponenten sowie (iii) der Detektionsprozesse für die Anwesenheit von spezifischen Amplifikationsprodukten nach der eigentlichen PCR. Alle diese Ereignisse würden zu falschnegativen Ergebnissen führen.

In den meisten dieser Systeme verlaufen die Einzelprozesse der Nukleinsäureisolierung, der Amplifikation per PCR und der Detektion bereits in einem vorgefertigten und produktionsbedingt geschlossenen System, das neben der initialen Einschleusung des Probenmaterials keine weitere Zugabe von individuellen Reaktionskomponenten oder sonstige Eingriffe durch den Anwender in das sukzessive bzw. sequenzielle Reaktionsgeschehen innerhalb der "Kartusche" oder der Reaktionskammer ermöglicht ("Pipeline-Konzept"). Anders als bei der bisher üblichen Praxis der "generischen" Nukleinsäureisolierung aus dem zu untersuchenden Probenmaterial über manuelle oder (teil-)automatisierte Isolierungs- und Aufreinigungsverfahren zur anschließenden Komplettierung von individuellen erregerspezifischen PCR-Reaktionsansätzen, wird die Flexibilität der Bereitstellung bzw. der Verwendung von individuellen PCR-Reaktionsgemischen bei geschlossenen Systemen zunehmend eingeschränkt. Es hat sich jedoch gezeigt, dass für einige Anwendungen (z.B. für Spezialuntersuchungen ohne große zu erwartende Serienzahlen) eine hohe Flexibilität vorteilhaft oder sogar notwendig ist, um Antworten auf diagnostische Fragestellungen zu erhalten, die mit den kommerziell erhältlichen geschlossenen PCR-Systemen nicht zu beantworten sind. Da gemäß der Richtlinie der Bundesärztekammer (RiliBÄK) B3 "Direkter Nachweis und Charakterisierung von Infektionserregern" interne Standards als positive und (bei nicht geschlossenen, vollautomatisierten Systemen) auch negative Kontrollprobe gefordert sind, besteht die Notwendigkeit neben der Probe zusätzliche Substanzen in ein geschlossenes PCR-System einschleusen zu können.

Es besteht daher ein Bedarf an PCR-Systemen, mit der auch diese Fragestellungen außerhalb des vorkonfektionierten Einsatzspektrums der produktionsbedingt geschlossenen PCR-Konzepte flexibel und zeitnah beantwortet werden können. Ein wesentliches Problem ist jedoch, dass in geschlossenen PCR-Systemen der Anwender außer dem Probenmaterial der Reaktionsmischung keine weiteren Reagenzien zuführen kann. So ist es beispielsweise unmöglich, die für die PCR notwendigen Primer- und/oder Sonden (-Oligonukleotide) zusammen mit dem Probenmaterial in das geschlossene System einzuschleusen, da sie konzeptbedingt den initialen Prozess der Nukleinsäureisolierung durchlaufen müssen. Dieser basiert in der Praxis meist auf der Lyse von (zu analysierenden) Zielorganismen und nachfolgender Aufreinigung von DNA- und RNA-Molekülen durch selektive Bindung der freigesetzten Nukleinsäuremoleküle an (modifizierten) Silikatoberflächen o.ä.. Während (langkettige) DNA- und RNA-Moleküle aufgrund ihrer hohen Bindungsaffinität gegenüber diesen Oberflächen an der Festphase gebunden bleiben, werden bei den üblicherweise durchgeführten Waschschritten störende Proteine und andere zelluläre Bestandteile ausgewaschen. Erst in einem folgenden Schritt werden (z.B. durch Änderung der Salz- oder pH-Bedingungen) die DNA- und RNA-Moleküle mit einem Elutionspuffer von Festphase selektiv eluiert. Dieser Schritt dient somit einerseits der Reinigung der DNA und RNA Moleküle und kann andererseits auch dazu genutzt werden, die Konzentration dieser Moleküle für die nachfolgende PCR zu erhöhen. In einigen Systemen stellt diese feste Phase eine physische Barriere zwischen verschiedenen Teilen des Systems dar, die nur von DNA und RNA Molekülen durchquert werden kann.

Da die Effizienz der Bindung von DNA und RNA Molekülen an Silikatoberflächen wesentliche von der Länge der Nukleinsäuremoleküle (mit Poly-anionischem Charakter) abhängig ist, ist die Bindungsaffinität kurzer Oligonukleotide zu diesen Oberflächen stark reduziert. Die üblicherweise für PCR verwendeten Primer- und Sondenmoleküle liegen meist als 12- bis 25-mere Oligonukleotide vor, die somit zu kurz sind, um effizient an die Oberflächen der stationären Phase gebunden werden zu können. Dementsprechend ist es - falls diese Moleküle dem aufzureinigenden Probenmaterial direkt zugegebenen werden - nicht effektiv möglich, diese in ausreichender Menge in den Teil des Systems zu überführen, in dem die PCR stattfindet. Systembedingt kann das Ausmaß des zu erwartenden Verlustes der Primer- und/oder Sondenmoleküle während der Aufreinigung nicht abgeschätzt werden, da dies u.a. von der individuellen Konsistenz sowie dem Gesamt-Nukleinsäuregehalt des biologischen Untersuchungsmaterials abhängig ist. Ein kalkulierter Ausgleich des Verlustes und somit die Möglichkeit einer gezielten "Überdosierung" ist daher in der Praxis nicht möglich.
Kommerziell erhältliche geschlossene PCR Systeme weisen daher meist bereits ein vorbestimmtes Primerpaar oder einen Primermix in dem Bereich auf, in dem die PCR stattfinden soll. Halbgeschlossene Systeme weisen einen separaten Zugang auf, über den Primer in den Bereich, in dem die PCR stattfinden soll, eingebracht werden können. Derartige Systeme mit einem zusätzlichen Zugang erfüllen jedoch nicht die mittlerweile sehr hohen Anforderungen in Bezug auf die Verhinderung von Kontaminationen und damit der Reproduzierbarkeit der Ergebnisse.

Aufgabe der Erfindung ist es daher, eine Möglichkeit zur Verwendung modifizierter Oligonukleotide in derartigen Systemen bereitzustellen, sowie ein Verfahren aufzuzeigen mit der das Einschleusen eines individuell gewählten Primers/Primerpaares und/oder von Sonden in den Bereich geschlossener PCR-Systeme, in dem die PCR stattfinden soll, ermöglicht wird.

Gelöst wird diese Aufgabe durch die Verwendung eines modifizierten Oligonukleotids gemäß Anspruch 1, ein Verfahren gemäß Anspruch 7 und eines Kits gemäß Anspruch 11.

Die erfindungsgemäße Verwendung eines modifizierten Oligonukleotids zeichnet sich dadurch aus, dass dieses mindestens eine Sonde und/oder einen PCR-Primer vorgegebener Sequenz umfasst und dass die Sonde/n (5) und/oder der/die PCR-Primer mittels mindestens eines spaltbaren Molekülteils mit mindestens einem Transporthelfer-Molekülteil verbunden ist, wobei durch den Transporthelfer-Molekülteil alleine oder in Kombination mit übrigen Teilen des modifizierten Oligonukleotids die Eigenschaften des modifizierten Oligonukleotids so eingestellt ist, dass es wie langkettige DNA- oder RNA-Moleküle aufgrund einer hohen Bindungsaffinität gegenüber einer Festphase zumindest temporär an deren Oberflächen bindbar und wie langkettige DNA- oder RNA-Moleküle von der Festphase eluiertbar ist, wobei das modifizierte Oligonukleotid zumindest zeitweise gemeinsam mit der Proben-DNA oder RNA auf dieser festen Phase gebunden wird, während übrige in der Probe vorhandene Substanzen in der wässrigen Phase verbleiben und in einem Reinigungsschritt zusammen mit Flüssigkeit entfernt werden und nach dieser Reinigung in einem Elutionsschritt das Lösen des modifizierten Oligonukleotids gemeinsam mit der Proben-DNA oder RNA von der festen Phase und das Einschleusen in den Bereich des geschlossenen PCR-Systems, in dem die PCR stattfinden soll, erfolgt und anschließend der/die Primer und/oder Sonde/n (5) der gewünschte Sequenz durch Spaltung des spaltbaren Molekülteil (4) von dem mindestens einen Transporthelfer-Molekülteil (3) freigesetzt wird/werden.

Durch diese Verwendung eines modifizierten Oligonukleotids ist es möglich, die für die vorgesehene/definierte PCR-Reaktion benötigten Oligonukleotidkomponenten, wie z.B. Primer (mit spezifischer Sequenz) und/oder eine bekannte Nukleinsäuresequenz zur Kontrolle des PCR-Verlaufs und/oder PCR-Sonden (z.B. Fluoreszenzmarkierungen für real-time PCR, Chromogene oder andersartige Detektions-Modifikationen, o.ä.) über den spaltbaren Molekülteil mit einem Transporthelfer-Molekülteil zu verbinden. Der Transporthelfer-Molekülteil (alleine oder in Kombination mit übrigen Teilen des modifizierten Oligonukleotids) sorgt dafür, dass die Eigenschaften des modifizierten Oligonukleotids so eingestellt werden können, dass es ähnlich wie langkettige DNA- oder RNA-Moleküle aufgrund einer hohen Bindungsaffinität gegenüber einer (Silikat-) Festphase (temporär) an deren Oberflächen gebunden werden kann. Genauso wie die langkettigen DNA- oder RNA-Moleküle kann auch das modifizierte Oligonukleotid von der Festphase eluiert werden, so dass es in den Bereich geschlossener PCR-Systeme, in dem die PCR stattfinden soll, eingeschleust werden kann.

Der Transporthelfer-Molekülteil sollte dabei so ausgewählt sein, dass er die Eigenschaften des Oligonukleotids so modifiziert, dass es einerseits unter ähnlichen Bedingungen wie langkettige DNA oder RNA Moleküle auf der festen Phase (temporär) fixiert wird und andererseits auch unter ähnlichen Bedingungen wie langkettige DNA- oder RNA-Moleküle von dieser eluiert werden kann. Als besonders geeignet haben sich dafür Polymere herausgestellt. In einer bevorzugten Ausführungsform ist daher der Transporthelfer-Molekülteil ein Polymer. Als geeignete Polymere kommen insbesondere Polymere in Betracht, die zumindest in Bezug auf eine Eigenschaft Ähnlichkeit zu DNA oder RNA aufweisen. Ähnlichkeiten können beispielsweise in Bezug auf die Anzahl von Aromaten (z.B. Polystyren), Kettenlänge, Molekülmasse, Polarität, Ladung oder anderer Moleküleigenschaften bestehen. Ein Fachmann ist in der Lage, einen Transporthelfer-Molekülteil auszuwählen, der sich unter den Bedingungen die zum Einschleusen der langkettigen DNA- oder RNA-Moleküle genutzt wird, ausreichend ähnlich verhält, so dass auch das modifizierte Oligonukleotid zusammen mit dem Transporthelfer-Molekülteil in den Bereich geschlossener PCR-Systeme, in dem die PCR stattfinden soll, eingeschleust werden kann.

Besonders große Ähnlichkeiten zu langkettigen DNA- oder RNA-Moleküle bestehen selbstverständlich dann, wenn der Transporthelfer-Molekülteil selbst ebenfalls ein Oligo- oder Polynukleotid ist. In einer besonders bevorzugten Ausführungsform ist daher der Transporthelfer-Molekülteil ein Oligo- oder Polynukleotid. Weiter bevorzugt ist die Länge des Oligo- oder Polynukleotids so gewählt, dass die Eigenschaften des modifizierten Oligonukleotids in dessen Gesamtheit im Wesentlichen den Eigenschaften der langkettigen DNA oder RNA Moleküle entsprechen, wie sie in der Probe vorliegen oder zu erwarten sind. So kann besonders gut gewährleistet werden, dass das modifizierte Oligonukleotid zusammen mit der langkettigen DNA oder RNA der Probe in den Bereich geschlossener PCR-Systeme, in dem die PCR stattfinden soll, eingeschleust werden kann. Bei dem Transporthelfer-Molekülteil kann es sich sowohl um einzel- als auch um doppelsträngige DNA oder RNA handeln.

Um zu verhindern, dass die Primer in dieser Ausführungsform mit dem Oligo- oder Polynukleotid des Transporthelfer-Molekülteils wechselwirken und letzteres während der PCR unerwünschter Weise amplifiziert wird, wird bevorzugt als Transporthelfer-Molekülteil ein Oligo- oder Polynukleotid verwendet, dessen Sequenz bekannt ist und bevorzugt keine ausreichend starke Hybridisierung mit der Sequenz des/der Primer eingeht, die dazu führen könnte, dass das Oligo- oder Polynukleotid des Transporthelfer-Molekülteils amplifiziert wird. So kann gewährleistet werden, dass im Wesentlichen nur die langkettigen DNA oder RNA Moleküle der Probe amplifiziert werden. Da artifizielle DNA- oder RNA-Oligo- oder Polynukleotide vergleichsweise teuer sind, bietet es sich an, dass das Oligo- oder Polynukleotid des Transporthelfer-Molekülteils eines natürlichen Ursprungs ist. Beispielsweise könne das Oligo- oder Polynukleotid des Transporthelfer-Molekülteils plasmidischen Ursprungs sein. Plasmidische DNA ist besonders leicht in ausreichend großen Mengen zu erhalten und vergleichsweise günstig. Darüber hinaus ist die Sequenz vieler Plasmide bekannt, so dass das Plasmid leicht so ausgewählt werden kann, dass es keine ausreichend starke Hybridisierung mit der Sequenz des/der Primer/Sonden(n) eingeht. Bevorzugt ist daher der Transporthelfer-Molekülteil ein Gen oder ein Teil eines Gens, dessen Sequenz bekannt ist und unter PCR-Bedingungen keine Hybridisierung mit der Sequenz des PCR-Primers oder der Sonde ausbildet.

In einer weiteren bevorzugten Ausführungsform kann der Transporthelfer-Molekülteil auch gleichzeitig eine Sonde sein oder umfassen. Beispielsweise könnte der Transporthelfer-Molekülteil eine Nukleinsäuresequenz umfassen, die während der PCR als Kontrolle fungiert und während der PCR ebenfalls amplifiziert wird. So könnte zusammen mit einem/einer oder mehreren/mehrerer Primer/n und/oder Sonde/n auch eine zusätzliche langkettige Nukleinsäuresequenz in den Bereich eingeschleust werden, in dem die PCR stattfinden soll, die als Kontrolle bzw. internen Standard fungieren kann. Um die Amplifikation der Kontroll-Nukleinsäuresequenz zu ermöglichen, müssen entsprechende Primer vorhanden sein. Diese könnten -sofern für ein vorgegebenes PCR-System bzw. einen PCR-Chip immer die bestimmte Kontroll-Nukleinsäuresequenz verwendet wird, bereits werksseitig in dem Bereich vorgelegt werden, in dem die PCR stattfinden soll. Andererseits könnten auch diese Primer zusammen mit der Kontroll-Nukleinsäuresequenz oder einem anderen modifizierten Oligonukleotid in diesen Bereich eingeschleust werden. Eine unerwünschte Hybridisierung außerhalb des PCR-Bereichs wird z.B. während der Reinigung oder der Zelllyse üblicherweise durch sehr hohe Salzkonzentrationen oder andere chemische oder physikalische Bedingungen, die eine unerwünschte Kreuzhybridisierung der Nukleinsäuresegmente hinreichend unterbinden, unterbunden. Während oder vor der PCR (z.B. zum Zeitpunkt der Spaltung des modifizierten Oligonukleotids durch Spaltung des/der spaltbaren Molekülteils/e) wird eine eventuell auftretende unerwünschte Hybridisierung durch die hohen Temperaturen im Denaturierungsschritt unterbunden.

Bevorzugt ist der Transporthelfer-Molekülteil über den spaltbaren Molekülteil (auch als "Linker" bezeichnet) am 5'-Ende des/der einzuschleusenden Primers/Sonde gebunden. Dieser Linker sollte unter den Bedingungen, unter denen die Trennung der DNA oder RNA Moleküle aus der Probe erfolgt, stabil sein und erst in einem späteren Schritt, nach dem Einschleusen des gesamten modifizierten Oligonukleotids gezielt spaltbar sein. So kann der Primer (beispielsweise durch Verbindung mittels des Linkers mit langen einzel- oder doppelsträngigen (Dummy- oder Reinigungshilfen-) Nukleinsäurefragmenten) die zuvor beschriebenen generischen Reinigungsprozesse ohne signifikanten Verlust oder andere Funktionseinschränkungen überstehen und zusammen mit dem Nukleinsäure-Eluat anschließend in das PCR-Reaktionsgemisch gelangen. Bevorzugt erfolgt erst dann die Spaltung des Linkers. In einer bevorzugten Ausführungsform ist der spaltbare Molekülteil chemisch, physikalisch oder enzymatisch spaltbar.

Unter einem chemisch spaltbaren Molekülteil soll eine Molekülstruktur verstanden werden, die als Reaktion auf ein verändertes chemisches Umfeld eine Reaktion eingeht, die zu einer Trennung dieser Molekülstruktur führen. Die Trennung kann dabei innerhalb der Molekülstruktur erfolgen oder an dessen Randbereichen. Als verändertes chemisches Umfeld, durch das die Reaktion ausgelöst werden kann, steht jede chemische Veränderung zur Verfügung, sofern sie Kompatibel zu den übrigen Reaktionsbedingungen ist und die Probe, die Sonde und/oder den Primer nicht in dem Maße schädigt, dass die nachfolgenden Reaktion im Rahmen der PCR oder der Detektion beeinträchtigt würden. Es werden daher chemisch spaltbare Molekülteile bevorzugt, die unter vergleichsweise milden Bedingungen die Spaltungsreaktion eingehen. Insbesondere bieten sich Spaltungen aufgrund von pH-Wert-Veränderungen, an, da DNA über einen vergleichsweise weiten pH-Bereich stabil ist. Weiterhin könnten beispielsweise Silylverbrückte Linker nicht nur durch pH-Wert-Erhöhung sondern auch durch Fluridionen gespalten werden. Linker mit mindestens einer Silylether-Funktion sind daher bevorzugt. Außerdem können Linker durch die Anwesenheit von (Schwer-) Metallionen oder Metallorganylen gespalten werden. Linker, die eine Esterfunktion aufweisen, sind grundsätzlich auch geeignet, da auch diese durch eine pH-Wert-Erhöhung spaltbar sind. Da jedoch zumindest einige Ester auch durch Lipasen spaltbar sind, die insbesondere bei der Lyse von Zellen aus einer Probe austreten können, haben sich Ester als Linker zumindest für diese Anwendung mit einer gemeinsamen Aufarbeitung der Probe und der modifizierten Oligonukleotide als weniger geeignet herausgestellt.

Unter physikalischer Spaltung soll eine Spaltung verstanden werden, die durch eine Änderung der physikalischen Bedingungen hervorgerufen wird. Insbesondre sind Temperaturänderungen vorgesehen. Da in der PCR für den Denaturierungsschritt ohnehin eine erhöhte Temperatur von meist 94-96°C vorgesehen ist, ist es besonders vorteilhaft, wenn der spaltbare Molekülteil spätesten bei diesen Temperaturen die Spaltungsreaktion eingeht und das Primer- oder Sondenmolekül freisetzt. Selbstverständlich kann die Spaltung des spaltbaren Molekülteils bereits bei geringeren Temperaturen wie z.B. 75°C, 50°C oder sogar bereits bei 40°C einsetzen. Bevorzugt ist jedoch, dass der spaltbare Molekülteil den Schritt der Lyse der Probe übersteht, ohne die Spaltungsreaktion einzugehen.

Eine Alternative oder Ergänzung zur thermischen Spaltung ist beispielsweise die Spaltung durch Bestrahlung mit Strahlung einer bestimmten Wellenlänge. In diesem Fall wird die Spaltung des spaltbaren Molekülteils beispielsweise elektromagnetische Strahlung wie sichtbares Licht (einer bestimmten Wellenlänge) oder UV-Licht ausgelöst.

Als enzymatische Spaltung wird in diesem Zusammenhang jegliche Freisetzung des/der Primer/s und/ oder Sonde/n der gewünschte Sequenz durch eine enzymatische Spaltung von mindestens einem Teil des modifizierten Oligonukleotids von einem anderen Teil des modifizierten Oligonukleotids verstanden. Denkbar wäre dazu die Implementierung von "biologischen" Linker-Molekülen, die in Anwesenheit bestimmter Enzyme im vorgegebenen PCR-Reaktionsgemisch zerstört werden. Diese Enzyme könnten dann vor Beginn der PCR während des initialen Heizschritts selbst wieder zerstört bzw. irreversibel inaktiviert werden. Die zur Spaltung notwendigen Enzyme könnten schon vor dem Einschleusen der Probe in dem Bereich des PCR-Systems, in dem die PCR stattfinden soll vorhanden sein, so dass sie nicht in diesen Bereich eingeschleust werden müssten. Eine besonders bevorzugte Ausführungsform zur enzymatischen Spaltung schließt Restriktionsendonukleasen ein, wobei insbesondere Typ II Endonukleasen bevorzugt sind. Besonders bevorzugt sind weiterhin Endonukleasen die "blunt ends", also "stumpfe Enden" erzeugen, da dadurch sie Anlagerung der getrennten Fragmente während der PCR vermieden wird. Bei der Verwendung von Restriktionsendonukleasen zum spalten des/der Linker(s) ist jedoch zu beachten, dass auch eine Spaltung in der Proben-DNA erfolgen kann und das Ergebnis der PCR somit verfälscht werden könnte. Daher sind Restriktionsendonukleasen bevorzugt, die eine Erkennungssequenz haben, die in der Proben-DNA nicht vorkommt oder deren Vorkommen dort zumindest sehr unwahrscheinlich ist.

In einer weiteren bevorzugten Ausführungsform der Verwendung des modifizierten Oligonukleotids weist das modifizierte Oligonukleotid (2) eine Vielzahl Molekülteilen auf, die ausgewählt sind aus der Gruppe, die Sonden (5) und PCR-Primer (5) umfasst. Durch diese Ausführungsform ist es möglich, möglichst alle während der PCR zusätzlich zur langkettigen DNA oder RNA der Probe erwünschten Moleküle in einem einzigen Molekül zu vereinen und diese gemeinsam in bevorzugt äquimolaren Konzentrationen in den Bereich des PCR-Systems einzuschleusen, in dem die PCR stattfinden soll. Selbstverständlich könnten auch andere molare Verhältnisse verschiedener, gemeinsam durch ein modifiziertes Oligonukleotid eingeschleuster Moleküle eingestellt werden. Beispielsweise könnte ein modifiziertes Oligonukleotid zwei Moleküle einer Sonde oder eines PCR-Primer vorgegebener Sequenz und ein Molekül einer anderen Sonde, eines Kontrollmolekülteils oder eines anderen PCR-Primers vorgegebener Sequenz umfassen. Derartige langkettige Moleküle mit einer Vielzahl von Funktionalitäten sind vergleichsweise teuer. Daher ist diese Ausführungsform nicht zwingend sondern es könnte - insbesondere um günstiger Moleküle einsetzen zu können - auch ein Mix aus verschiedenen Molekülen verwendet werden, die beispielsweise nur einen Transporthelfer-Molekülteil, einen spaltbaren Molekülteil und genau eine Sonde oder PCR-Primer aufweisen. Analog sind selbstverständlich auch modifizierte Oligonukleotide denkbar, die nur einen Transporthelfer-Molekülteil, einen spaltbaren Molekülteil und genau zwei Molekülteile aufweisen die ausgewählt sind aus der Gruppe, die Sonden und PCR-Primer umfasst. Insbesondere wäre es beispielsweise denkbar, (FRET-) Sondenpaare oder Primerpaare (Forward-Primer und Reverse-Primer) in jeweils modifiziertes Oligonukleotid zu integrieren und so gemeinsam in jeweils zwangsläufig gleicher Konzentration in den PCR-Bereich einschleusen zu können.

In einer weiteren bevorzugten Variante zur Verwendung des modifizierten Oligonukleotids ist vorgesehen, dass mindestens eine Sonde über eine Kupplungsstelle mit einen spaltbaren Molekülteils verbunden ist, die nach der Spaltung des spaltbaren Molekülteils unter PCR-Bedingungen nicht durch Polymerasen verlängerbar ist. Dies lässt sich vergleichsweise einfach realisieren, da üblicherweise die PCR am 3'-Ende des Templats eine freie Hydroxygruppe erfordert. Jeglicher spaltbaren Molekülteil (Linker) der nach seiner Spaltung an der Sonde eine andere (PCR-kompatible, also die PCR-Reaktion und insbesondere die Polymerase nicht hemmende) Gruppe hinterlässt, bietet diese Möglichkeit. Als mögliche am (3'-) Ende der Sonde verbleibende funktionelle Gruppe kommen insbesondere Aldehyd-, Alkyl-, Alkylen-, Amin-, Ether-, Hydrazin, Imin-, Keton-, Nitril-, Thioaldehyd-, Thioether-, Thioketon- und Thiol-Reste denkbar. Ein Fachmann ist in der Lage, die Kompatibilität der mit der jeweiligen Endgruppe versehenen Sonde entsprechend den jeweiligen PCR-Bedingungen auszuwählen.

Ein weiterer Aspekt der Erfindung ist ein Verfahren zum Einschleusen eines Primers und/oder einer Sonde in ein geschlossenes PCR-System, bei dem zunächst ein modifiziertes Oligonukleotid ausgewählt wird, das mindestens eine Sonde und/oder einen Primer einer gewünschten Sequenz sowie mindestens einen spaltbaren Molekülteil und mindestens einen Transporthelfer-Molekülteil umfasst, wobei dieses Transporthelfer-Molekülteil sich in seiner Affinität zu einer festen Phase wie eine zu amplifizierende DNA oder RNA der Probe verhält, um das modifizierte Oligonukleotid zumindest zeitweise gemeinsam mit der Proben-DNA oder RNA auf dieser festen Phase zu binden, während übrige in der Probe vorhandene Substanzen in der wässrigen Phase verbleiben und in einem Reinigungsschritt zusammen mit Flüssigkeit entfernt werden und nach dieser Reinigung in einem Elutionsschritt das Lösen des modifizierten Oligonukleotids gemeinsam mit der Proben-DNA oder RNA von der festen Phase und das Einschleusen in den Bereich des geschlossenen PCR-Systems, in dem die PCR stattfinden soll, erfolgt und anschließend der/die Primer und/oder Sonde/n (5) der gewünschte Sequenz durch Spaltung des spaltbaren Molekülteil (4) von dem mindestens einen Transporthelfer-Molekülteil (3) freigesetzt wird/werden.

Es ist somit vorgesehen, dass dieses modifizierte Oligonukleotid als Gesamtheit durch eine Schleuse des geschlossenen PCR-Systems geschleust wird und anschließend der/die Sonde/n und/oder Primer der gewünschten Sequenz durch Spaltung des spaltbaren Molekülteils von dem mindestens einen Transporthelfer-Molekülteil freigesetzt werden. Auf diese Weise wird die Herstellung von kommerziell vorgefertigten geschlossenen "generischen PCR-Kartuschen" möglich, die vom Anwender, je nach aktueller Fragestellung, durch die Zugabe von individuellen "Primer- und/oder Sonden-Oligonukleotid-Linker-Transporthelfer-Molekülteil-Oligonukleotide" direkt zum Probenmaterial flexibel, aber im Gesamtkonzept und Analyseablauf dennoch standardisiert, eingesetzt werden können.

Durch die Verbindung des/der Primer und/oder Sonde/n der für die PCR benötigten Sequenz über den spaltbaren Molekülteil mit dem Transporthelfer-Molekülteil wird es möglich, dass der/die Primer und/oder Sonde/n ähnlich wie DNA oder RNA durch eine Barriere, die für andere Substanzen z.B. eines Zelllysats wie z.B. Proteine, Zellmembranfragmente u.a. undurchlässig ist, geschleust werden kann. Bevorzugt kann so sogar der/die Primer und/oder Sonde/n zusammen mit der DNA oder RNA in den Bereich des geschlossenen PCR-Systems geschleust werden. Dadurch kann das Verfahren weiter vereinfacht und weitere Fehlerquellen beseitigt werden. Bevorzugt wird daher das modifizierte Oligonukleotid gemeinsam mit der Probe, die die per PCR zu amplifizierende DNA enthält, durch eine Schleuse des geschlossenen PCR-Systems geschleust.

Eine weitere Vereinfachung ergibt sich, wenn das modifizierte Oligonukleotid die Bedingungen, unter denen die Probe für die PCR vorbereitet wird (z.B. eine Zelllyse) ebenfalls unbeschadet überstehen kann. Dies stellt insbesondere Ansprüche an den spaltbaren Molekülteil des modifizierten Oligonukleotids, da dieser nicht unter den Reinigungsbedingungen gespalten werden darf. Daher sollte bevorzugt die Probenaufreinigung und/oder Probenvorbereitung unter Bedingungen erfolgen, in denen der als Linker verwendete spaltbare Molekülteil nicht gespalten wird. Diese Bedingungen können je nach Auswahl des spaltbaren Molekülteils einen bestimmten Temperaturbereich (z.B. < 40°C), einen bestimmten pH-Bereich (z.B. pH 6 - 8), Lichtausschluss, Ausschluss von elektromagnetischer Strahlung einer bestimmten Wellenlänge, Abwesenheit von bestimmten Anionen (z.B. F⁻), Abwesenheit von bestimmten Metallionen und andere einschließen. In einer bevorzugten Ausführungsform wird daher das modifizierte Oligonukleotid gemeinsam mit der Probe, die die per PCR zu amplifizierende DNA enthält, mindestens einem Reinigungsschritt unterzogen.

Weiter bevorzugt ist, dass der/die Primer und/oder Sonde/n der gewünschten Sequenz nach dem Einschleusen in einen Bereich des geschlossenen PCR-Systems, in dem die PCR stattfinden soll, durch chemische, physikalische oder enzymatische Spaltung des spaltbaren Molekülteils von dem mindestens einen Transporthelfer-Molekülteil freigesetzt wird/werden. Bevorzugt wird der/die Primer und/oder Sonde/n der gewünschten Sequenz durch thermische Spaltung des spaltbaren Molekülteils von dem mindestens einen Transporthelfer-Molekülteil freigesetzt. Im Fall einer thermisch induzierten Freisetzung kann die Freisetzung vor oder während des Starts der PCR-typischen Thermozyklen durch Spaltung des (spaltbaren) Linker-Moleküls von dem Transporthelfer-Molekülteil erfolgen. So stehen sie unmittelbar und funktionell als Primer- und/oder Sondenmoleküle für den spezifischen PCR-Prozess zur Verfügung. In einer weiter bevorzugten Verfahrensvariante erfolgt die thermische Spaltung des spaltbaren Molekülteils während eines Denaturierungsschritts der PCR-Reaktion bei 94-96°C.

Der Transporthelfer-Molekülteil ist bevorzugt so ausgewählt dass er sich in seiner Affinität zu einer festen Phase ähnlich wie die zu amplifizierende (langkettige) DNA oder RNA der Probe verhält. Insbesondere ist in einer bevorzugten Ausführungsform vorgesehen, dass das modifizierte Oligonukleotid zumindest zeitweise auf einer festen Phase gebunden wird. Dadurch wird ermöglicht, dass das modifizierte Oligonukleotid gemeinsam mit der Proben-DNA oder RNA auf dieser festen Phase gebunden ist während übrige in der Probe vorhandene Substanzen wie Proteine, Zellwandfragmente u.ä. in der wässrigen Phase verbleiben und in einem Reinigungsschritt zusammen mit Flüssigkeit entfernt werden können. Während möglicher anschließender weiterer zusätzlicher Reinigungsschritte kann das modifizierte Oligonukleotid gemeinsam mit der Proben-DNA oder RNA auf der festen Phase gebunden bleiben. Nach erfolgter Reinigung erfolgt in einem Elutionsschritt das Lösen des modifizierten Oligonukleotids gemeinsam mit der Proben-DNA oder RNA von der festen Phase und (evtl. zeitgleich) das Einschleusen in den Bereich des geschlossenen PCR-Systems, in dem die PCR stattfinden soll. Das Design des Transporthelfer-Molekülteils ist dabei bevorzugt so gewählt, dass die spezifische PCR durch die Anwesenheit des Transporthelfer-Molekülteils nicht gestört oder beeinträchtigt wird. Als besonders bevorzugt hat sich daher herausgestellt als Transporthelfer-Molekülteil Nukleinsäurefragmente zu verwenden, die keine für eine zur Kettenverlängerung während der PCR ausreichende Hybridisierung aufgrund von Komplementarität zu den Sonden- und/oder Primer-Oligonukleotiden aufweisen. Als besonders bevorzugt hat sich herausgestellt, Nukleinsäurefragmente bekannter Sequenz (z.B. eines einzelnen Gens oder eines Plasmids) zu verwenden.

Weiterhin betrifft die Erfindung ein Kit das das Einschleusen von Primer/n und/oder Sonde/n in ein geschlossenes PCR-System ermöglicht, wobei das Kit ein wie oben beschriebenes modifiziertes Oligonukleotid sowie mindestens ein Auxiliar zur Freisetzung des/der Primer/s und/oder Kontrollmoleküls/e und/oder Sonde/n aus diesem modifizierten Oligonukleotid umfasst. Als Auxiliar wird in diesem Zusammenhang jeglicher Hilfsstoff verstanden, der gemeinsam mit dem modifizierten Oligonukleotid oder getrennt von diesem in den Bereich des PCR-Systems, in dem die PCR stattfinden soll, eingeschleust werden kann und dort eine Freisetzung des/der Primer/s und/oder Sonde/n aus diesem modifizierten Oligonukleotid hervorruft und/oder unterstützt. Beispielsweise könnte ein Auxiliar nach der Einschleusung in Folge einer externen Anregung (z.B. Temperaturerhöhung oder Bestrahlung) den pH-Wert verändern, so dass eine Spaltung des Linkers eintritt.

Weiterhin offenbart ist ein Kit, das neben einem modifizierten Oligonukleotid auch ein PCR-System mit einer für das modifizierte Oligonukleotid und optional auch das Auxiliar kompatiblen Schleuse umfasst.

Weitere Vorteile und Zweckmäßigkeiten sind exemplarisch anhand der nachfolgenden Beschreibung einer bevorzugten Ausführungsform in Verbindung mit den Figuren zu entnehmen. Hierbei zeigen:
- Fig. 1: eine schematische Darstellung eines Ausführungsbeispiels der vorliegenden Erfindung;

- Fig. 2: eine ausschnittsweise Darstellung des humanen β-Globin-Gens und geeigneter Primer zur Amplifizierung bestimmter Segmente;
- Fig. 3: eine grafische Darstellung der durch Amplifizierung erhaltenen PCR-Produkte;
- Fig. 4: einen Vergleich der Amplifizierung von bestimmten Segmenten des β-Globin-Gens aus verschiedenen Proben gegenüber einer negativen Kontrollprobe.

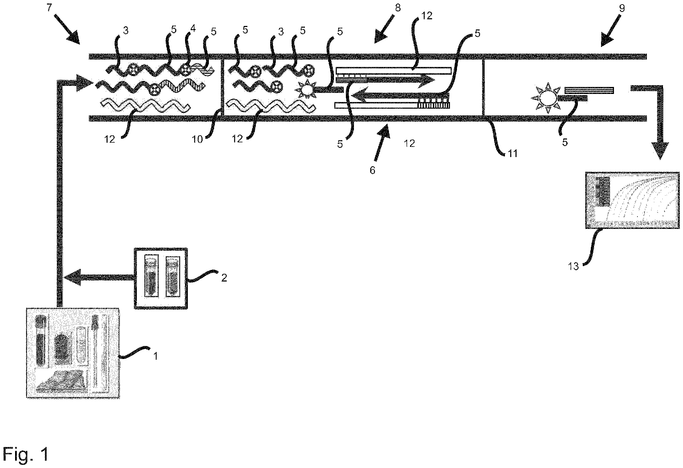

Figur 1 zeigt eine schematische Darstellung eines Ausführungsbeispiels der vorliegenden Erfindung. Mit Bezugszeichen 1 ist das Proben- oder Untersuchungsmaterial gekennzeichnet. Hierbei kann es sich beispielsweise um Blut, Haut, Haare, Speichel oder jegliches andere Probenmaterial handeln. Diesem kann nach oder während der Aufbereitung ein oder mehrere modifizierte Oligonukleotid(e) 2 zugesetzt werden. Ein solches modifiziertes Oligonukleotid 2 umfasst einen Transporthelfer-Molekülteil 3, einen spaltbaren Molekülteil 4 und einen Primer oder ein Kontrollmolekül oder eine Sonde, die unabhängig von ihre Funktion jeweils mit dem Bezugszeichen 5 gekennzeichnet sind. Wie an einem Beispiel exemplarisch dargestellt ist, können die modifizierten Oligonukleotide auch jeweils mehrere funktionelle Molekülteile wie Primer und/oder Kontrollmoleküle und/oder Sonden enthalten.

Das PCR-System 6 ist in Figur 1 lediglich schematisch als Rohr dargestellt. Es ist in drei verschiedene Bereiche 7, 8 und 9 unterteilt, die jeweils durch geeignete Trenneinrichtungen 10, 11 voneinander getrennt sind. Der mit dem Bezugszeichen 7 gekennzeichnete Bereich stellt einen Probenaufbereitungsbereich 7 dar. In diesem kann die Probe für die PCR vorbereitet werden. Beispielsweise könnte hier die Zelllyse stattfinden. Im gezeigten Beispiel sind im Probenaufbereitungsbereich 7 sowohl modifizierte Oligonukleotide 2 als auch Proben-DNA 12 gezeigt. Es ist jedoch nicht zwingend notwendig, dass in diesem Bereich sowohl modifizierte Oligonukleotide 2 als auch Proben-DNA 12 gleichzeitig vorhanden sind. Es wäre ebenfalls möglich, zuerst eine dieser Substanzen im Probenaufbereitungsbereich 7 zu behandeln und durch die Trenneinrichtung 10 in den PCR-Bereich 8 zu überführen, bevor die nächste Substanz in den Probenaufbereitungsbereich 7 gelangt und anschließend ebenfalls in den PCR-Bereich 8 überführt wird.

Der PCR-Bereich 8 ist der Bereich, in dem die eigentliche PCR und damit die Amplifizierung der Proben-DNA 12 stattfindet. Da es sich um ein geschlossenes System handelt, ist der PCR-Bereich 8 allseitig geschlossen, wobei die Trenneinrichtungen 10, 11 für bestimmte Substanzen wie insbesondere Proben-DNA 12 und modifizierte Oligonukleotide 2 zumindest temporär durchlässig sind. Der PCR-Bereich 8 kann durch nicht gezeigte Temperiereinrichtungen auf bestimmte Temperaturen eingestellt werden. Wie bei der PCR üblich ist ein schnelles Temperieren der gesamten Probe vorteilhaft. Die Temperiereinrichtungen können daher neben Heizeinrichtungen auch Kühleinrichtungen aufweisen. In den meisten kommerziell erhältlichen geschlossenen PCR-Systemen ist der PCR-Bereich 8 als in Kurven verlaufende Leitung ausgebildet, die wechselweise heiße und kalte Bereiche durchläuft. Beim Durchleiten der PCR-Lösung erwärmt sich diese in Abhängigkeit von der aktuellen Position oder kühlt sich ab.

Im PCR-Bereich 8 erfolgt vor oder zusammen mit dem Start der PCR eine Spaltung des modifizierten Oligonukleotids 2. Dies kann beispielsweise durch thermische Spaltung des Linkers 4 erfolgen. Dies hat sich als besonders geeignet erwiesen, da im PCR-Bereich 8 ohnehin eine Temperiereinrichtung vorhanden sein muss und so die Spaltung des/der spaltbaren Molekülteils/e 4 apparativ besonders einfach zu realisieren ist. Um dem System für eine möglichst fehlerfreie Anlagerung der Primer an die komplementären DNA-Bereiche zu ermöglichen, bietet es sich an, das System für ein längeres initiales Zeitintervall bei erhöhter Temperatur zu halten. Während dieser Zeit von z.B. 10 Minuten bei 95°C kann auch die Spaltung des/der spaltbaren Molekülteils/e 4 und damit die Freisetzung des/der Primer/s 5 aus dem modifizierten Oligonukleotid 2 erfolgen.

Nach der Durchführung der PCR werden die PCR-Produkte durch die Trenneinrichtung 11 aus dem PCR-Bereich 8 ausgeschleust. Die Trenneinrichtung 11 kann dabei wesentlich weniger selektiv als die Trenneinrichtung 10 sein, so dass auch kürzere DNA-Fragmente passieren könnten. In dem sich an den PCR-Bereich 8 anschließenden Detektionsbereich 9 erfolgt beispielsweise die Detektion von speziellen Amplifikationsprodukten. Dies kann beispielsweise mittels Sonden 5 erfolgen. Alternativ oder zusätzliche wäre auch die Detektion bereits im PCR-Bereich 8 beispielsweise im Rahmen einer "real-time-PCR" möglich. Anschließend kann das Ergebnis ausgewertet und/oder auf einer Anzeigeeinrichtung 13 ausgegeben werden.

Als beispielhaftes Modellsystem für die Durchführung von diagnostischen PCR-Untersuchungen in der medizinischen Mikrobiologie bietet sich die Darstellung eines spezifischen Nachweises des humanen β-Globin-Gens im Rahmen einer semiquantitativen real-time PCR an. Humane Gensequenzen dienen bei der akkreditierungskonformen Durchführung von diagnostischen PCR-Untersuchungen in der medizinischen Mikrobiologie häufig als interne oder externe Extraktionskontrollen zur Bestätigung eines korrekten Ablaufs der Nukleinsäureisolierung aus klinischem Probenmaterial. Eine solche Sequenz könnte jedoch auch beispielsweise als Transporthelfer-Molekülteil 3 und optional sogar gleichzeitig als ein Kontrollmolekülteil 5 eines modifizierten Oligonukleotids 2 genutzt werden. Durch die Länge einer solchen Sequenz ist sie in der Lage, einerseits in der Wirkung als Transporthelfer-Molekülteil 3 die Eigenschaften des modifizierten Oligonukleotids 2 so zu verändern, dass es die Trenneinrichtung bzw. Schleuse 10 passieren kann, andererseits jedoch als interner Standard für die PCR dienen und somit als Kontrollmolekülteil 5 eines modifizierten Oligonukleotids 2 wirken.

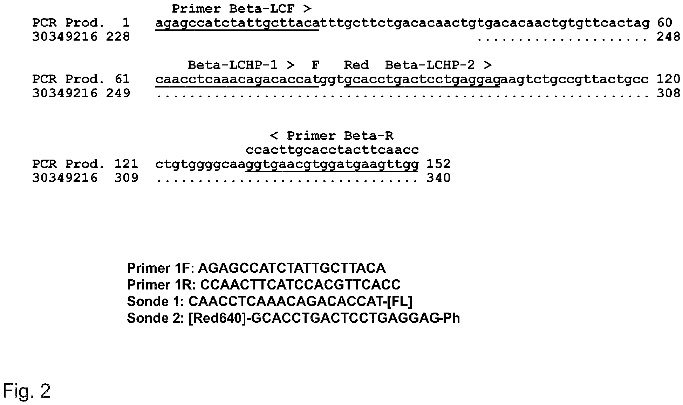

Figur 2 zeigt ausschnittsweise Darstellungen des humanen β-Globin-Gens und jeweils geeigneter Primer zur Amplifizierung bestimmter Segmente. Zwei Sequenzen sind FRET-Sonden und weisen entweder einen Donor oder Akzeptor eines FRET-Donor-Akzeptor-Paares auf. Durch diese ist es im Rahmen einer Routinediagnostik möglich, eine erfolgreiche Extraktion von DNA aus klinischem Probematerial zu belegen. Die verwendeten Primer und Sonden weisen die jeweils folgenden Sequenzen auf:
Primer 1F: AGAGCCATCTATTGCTTACA pos. 228-247 in AY26074)
Primer 1R: CCAACTTCATCCACGTTCACC (pos. 340-320 in AY26074)
Sonde 1: CAACCTCAAACAGACACCAT-[FL] (pos. 249-268 in AY26074)
Sonde 2: [Red640]-GCACCTGACTCCTGAGGAG-Ph (pos. 272-290 in AY26074)

Die Sonden Sonde 1 und Sonde 2 bilden das FRET-Paar, mittels dem die Amplifikationskontrolle der real-time-PCR vorgenommen wird. Das durch die Primer Primer 1 und Primer 2 amplifizierbare Fragment des humanen β-Globin-Gens erstreckt sich von Position 228 bis Position 340. Die Gesamtsequenz des Amplifikats ist den in Figur 2 gezeigten Fragmenten zu entnehmen.

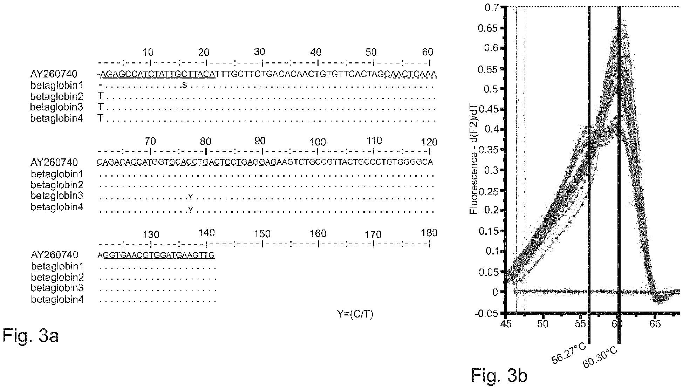

Ausgehend von homogenem humanem Probenmaterial (z.B. Blut) wurden vergleichende Experimente mit äquimolaren Mengen an (i) nativen Oligonukleotiden und (ii) Oligonukleotiden initial gekoppelt mit Transporthelfer-Molekülteil und anschließend abgespalten, im Rahmen von semiquantitativen real-time PCR Ansätzen durchgeführt. Dieser Ansatz kann auch dazu verwendet werden, die Effizienz der jeweiligen Abspaltungsprozesse von Reinigungshilfe-Nukleinsäuren bei Anwendung bestimmter Bedingungen systematisch zu untersuchen. Figur 3a zeigt eine grafische Darstellung der durch Amplifizierung erhaltenen PCR-Produkte. Sie ermöglichen einen Vergleich verschiedener β-Globin-Gene und die Detektion eventueller Mutationen. Im gezeigten Beispiel sind 4 Proben (als "betaglobin 1 - 4" bezeichnet) per PCR amplifiziert worden und das Schmelzverhalten der Produkte analysiert worden. Es konnte gezeigt werden, dass in den Proben betaglobin 3 und 4 Amplifikate gebildet werden, die sich an der Position Nr. 77 unterscheiden. Diese Position ist mit "y" gekennzeichnet. Durch diese Veränderung unterscheiden sich die Amplifikate im Schmelzpunkt, was in Figur 3b dargestellt ist. Die Amplifikate der Proben betaglobin 3 und 4 weisen daher jeweils 2 Schmelzpunkte auf, die bei etwa 56 und 60°C liegen.

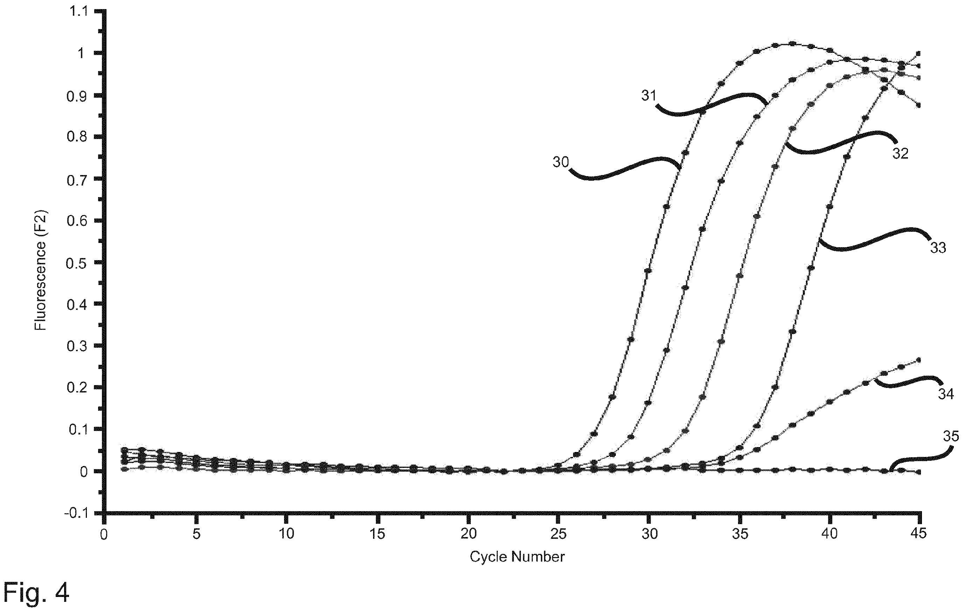

Figur 4 zeigt einen Vergleich der Amplifizierung von bestimmten Segmenten des β-Globin-Gens aus verschiedenen Proben gegenüber einer negativen Kontrollprobe. Die verschiedenen Proben entstammen einer humanen Gewebebiopsie (tissue biopsy) 30, einem Glaskörperpunktat des Auges (vitreous (GK)) 31, einer Vorderkammerpunktion des Auges (aqueous humor (VK)) 32, dem Liquor bzw. Zerebrospinalflüssigkeit (Cerebrospinal fluid (CSF)) 33 und dem Vorderkammerwassers des Auges (aqueous humor (VK)) 34. Die Probe 35 ist eine negative Kontrollprobe. Durch diese Experimente konnte gezeigt werden, dass unabhängig von der Herkunft der Probe 30 - 34 eine effiziente Amplifizierung der enthaltenen DNA möglich ist. Die negative Kontrollprobe 35 lieferte keine detektierbaren Amplifikate.

### Bezugszeichenliste

- 1: Proben- oder Untersuchungsmaterial
- 2: modifiziertes Oligonukleotid
- 3: Transporthelfer-Molekülteil
- 4: spaltbarer Molekülteil
- 5: Primer, Kontrollmolekül oder Sonde
- 6: PCR-System
- 7: Bereich
- 8: Bereich
- 9: Bereich, Detektionsbereich
- 10: Trenneinrichtung
- 11: Trenneinrichtung
- 12: Proben-DNA
- 13: Anzeigeeinrichtung
- 30: humane Gewebebiopsie (tissue biopsy)
- 31: Glaskörperpunktat des Auges (vitreous (GK))
- 32: Vorderkammerpunktion des Auges (aqueous humor (VK))
- 33: Liquor bzw. Zerebrospinalflüssigkeit (Cerebrospinal fluid (CSF))
- 34: Vorderkammerwasser des Auges (aqueous humor (VK))
- 35: negative Kontrollprobe

## Patentansprüche

1. Verwendung eines modifizierten Oligonukleotids (2), umfassend mindestens eine Sonde (5) und/oder einen PCR-Primer (5) vorgegebener Sequenz zum Einschleusen der mindestens einen Sonde (5) und/oder PCR-Primers in ein geschlossenes PCR-System,
**dadurch gekennzeichnet, dass**
ein Oligonukleotid (2) ausgewählt wird, welches mindestens die Sonde/n (5) und/oder der/die PCR-Primer (5) umfasst, welche mittels mindestens eines spaltbaren Molekülteils (4) mit mindestens einem Transporthelfer-Molekülteil (3) verbunden ist, wobei durch den Transporthelfer-Molekülteil alleine oder in Kombination mit übrigen Teilen des modifizierten Oligonukleotids die Eigenschaften des modifizierten Oligonukleotids so eingestellt ist, dass es wie langkettige DNA- oder RNA-Moleküle aufgrund einer hohen Bindungsaffinität gegenüber einer Festphase zumindest temporär an deren Oberflächen bindbar und wie langkettige DNA- oder RNA-Moleküle von der Festphase eluiertbar ist, wobei das modifizierte Oligonukleotid zumindest zeitweise gemeinsam mit der Proben-DNA oder RNA auf dieser festen Phase gebunden wird, während übrige in der Probe vorhandene Substanzen in der wässrigen Phase verbleiben und in einem Reinigungsschritt zusammen mit Flüssigkeit entfernt werden und
nach dieser Reinigung in einem Elutionsschritt das Lösen des modifizierten Oligonukleotids gemeinsam mit der Proben-DNA oder RNA von der festen Phase und das Einschleusen in den Bereich des geschlossenen PCR-Systems, in dem die PCR stattfinden soll, erfolgt und
anschließend der/die Primer und/oder Sonde/n (5) der gewünschte Sequenz durch Spaltung des spaltbaren Molekülteil (4) von dem mindestens einen Transporthelfer-Molekülteil (3) freigesetzt wird/werden.

2. Verwendung nach Anspruch 1,
**dadurch gekennzeichnet, dass**
der Transporthelfer-Molekülteil (3) ein Oligo- oder Polynukleotid, bevorzugt ein Gen oder ein Teil eines Gens ist, dessen Sequenz bekannt ist.

3. Verwendung nach Anspruch 2,
**dadurch gekennzeichnet, dass**
der Transporthelfer-Molekülteil (3) unter PCR-Bedingungen keine Hybridisierung mit der Sequenz des PCR-Primers (5) und/oder der Sonde (5) ausbildet.

4. Verwendung nach einem der vorangegangenen Ansprüche,
**dadurch gekennzeichnet, dass**
der spaltbare Molekülteil (4) chemisch, physikalisch oder enzymatisch spaltbar ist.

5. Verwendung nach einem der vorangegangenen Ansprüche,
**dadurch gekennzeichnet, dass**
das modifiziertes Oligonukleotid (2) eine Vielzahl Molekülteilen aufweist, die ausgewählt sind aus der Gruppe, die Sonden (5) und PCR-Primer (5) umfasst.

6. Verwendung nach einem der vorangegangenen Ansprüche,
**dadurch gekennzeichnet, dass**
mindestens eine Sonde (5) über eine Kupplungsstelle mit einem spaltbaren Molekülteil (4) verbunden ist, der nach der Spaltung des spaltbaren Molekülteils (4) unter PCR-Bedingungen nicht durch Polymerasen verlängerbar ist.

7. Verfahren zum Einschleusen eines Primers und/oder einer Sonde in ein geschlossenes PCR-System (6),
**dadurch gekennzeichnet, dass**
ein modifiziertes Oligonukleotid (2) ausgewählt wird, das mindestens eine Sonde (5) und/oder einen Primer (5) einer gewünschten Sequenz sowie mindestens einen spaltbaren Molekülteil (4) und mindestens einen Transporthelfer-Molekülteil (3) umfasst, wobei dieses Transporthelfer-Molekülteil sich in seiner Affinität zu einer festen Phase wie eine zu amplifizierende DNA oder RNA der Probe verhält, um das modifizierte Oligonukleotid zumindest zeitweise gemeinsam mit der Proben-DNA oder RNA auf dieser festen Phase zu binden, während übrige in der Probe vorhandene Substanzen in der wässrigen Phase verbleiben und in einem Reinigungsschritt zusammen mit Flüssigkeit entfernt werden und
nach dieser Reinigung in einem Elutionsschritt das Lösen des modifizierten Oligonukleotids gemeinsam mit der Proben-DNA oder RNA von der festen Phase und das Einschleusen in den Bereich des geschlossenen PCR-Systems, in dem die PCR stattfinden soll, erfolgt und
anschließend der/die Primer und/oder Sonde/n (5) der gewünschte Sequenz durch Spaltung des spaltbaren Molekülteil (4) von dem mindestens einen Transporthelfer-Molekülteil (3) freigesetzt wird/werden.

8. Verfahren nach Anspruch 7,
**dadurch gekennzeichnet, dass**
der/die Primer und/oder Sonde(n) (5) der gewünschten Sequenz durch chemische, physikalische oder enzymatische Spaltung des spaltbaren Molekülteils (4) von dem mindestens einen Transporthelfer-Molekülteil (3) freigesetzt wird/werden.

9. Verfahren nach einem der Ansprüche 7 oder 8,
**dadurch gekennzeichnet, dass**
der/die Primer und/oder Sonde(n) (5) der gewünschten Sequenz durch thermische Spaltung des spaltbaren Molekülteils (4) von dem mindestens einen Transporthelfer-Molekülteil (3) freigesetzt wird/werden.

10. Verfahren nach Anspruch 7,
**dadurch gekennzeichnet, dass**
die thermische Spaltung des spaltbaren Molekülteils (4) während eines Denaturierungsschritts der PCR-Reaktion, bevorzugt bei 94-96°C erfolgt.

11. Kit zum Einschleusen eines Primers (5) und/oder einer Sonde (5) in ein geschlossenes PCR-System (6),
**dadurch gekennzeichnet, dass**
das Kit ein modifiziertes Oligonukleotid (2) gemäß einem der Ansprüche 1 - 6 sowie mindestens ein Auxiliar zur Freisetzung des Primers (5) und/oder der Sonde (5) aus diesem modifizierten Oligonukleotid (2) umfasst.

## Claims

1. Use of a modified oligonucleotide (2) comprising at least one probe (5) and/or a PCR primer (5) of a specified sequence in order to feed the at least one probe (5) and/or PCR primer into a closed PCR system,
**characterised in that**
an oligonucleotide (2) is selected which comprises at least the probe(s) (5) and/or the PCR primer(s) (5) that is/are connected to at least one transport helper molecule part (3) by means of at least one cleavable molecule part (4), the properties of the modified oligonucleotide being set by the transport helper molecule part alone or in combination with remaining parts of the modified oligonucleotide such that, on account of a high binding affinity to a solid phase, it can be at least temporarily bound to the surfaces thereof like long-chain DNA or RNA molecules and can be eluted from the solid phase like long-chain DNA or RNA molecules, the modified oligonucleotide being bound at least temporarily together with the sample DNA or RNA to said solid phase, while remaining substances present in the sample remain in the aqueous phase and are removed together with liquid in a cleaning step, and
after this cleaning, in an elution step, the modified oligonucleotide is detached from the solid phase together with the sample DNA or RNA and fed into the region of the closed PCR system in which the PCR is intended to occur, and the primer(s) and/or probe(s) (5) of the desired sequence is/are subsequently released from the at least one transport helper molecule part (3) by cleaving the cleavable molecule part (4).

2. Use according to claim 1,
**characterised in that**
the transport helper molecule part (3) is an oligonucleotide or polynucleotide, preferably a gene or a part of a gene, of which the sequence is known.

3. Use according to claim 2,
**characterised in that**
the transport helper molecule part (3) does not form a hybridisation having the sequence of the PCR primer (5) and/or the probe (5) under PCR conditions.

4. Use according to any of the preceding claims,
**characterised in that**
the cleavable molecule part (4) can be chemically, physically or enzymatically cleaved.

5. Use according to any of the preceding claims,
**characterised in that**
the modified oligonucleotide (2) comprises a plurality of molecule parts which are selected from the group that comprises probes (5) and PCR primers (5).

6. Use according to any of the preceding claims,
**characterised in that**
at least one probe (5) is connected to a cleavable molecule part (4) via a coupling site, which molecule part cannot be lengthened by polymerases after the cleavable molecule part (4) has been cleaved under PCR conditions.

7. Method for feeding a primer and/or a probe into a closed PCR system (6), **characterised in that**
a modified oligonucleotide (2) is selected which comprises at least one probe (5) and/or one primer (5) of a desired sequence and at least one cleavable molecule part (4) and at least one transport helper molecule part (3), said transport helper molecule part behaving, in terms of the affinity thereof to a solid phase, like a DNA or RNA of the sample to be amplified in order to bind the modified oligonucleotide at least temporarily together with the sample DNA or RNA to said solid phase, while remaining substances present in the sample remain in the aqueous phase and are removed together with liquid in a cleaning step, and
after this cleaning, in an elution step, the modified oligonucleotide is detached from the solid phase together with the sample DNA or RNA and fed into the region of the closed PCR system in which the PCR is intended to occur, and the primer(s) and/or probe(s) (5) of the desired sequence is/are subsequently released from the at least one transport helper molecule part (3) by cleaving the cleavable molecule part (4).

8. Method according to claim 7,
**characterised in that**
the primer(s) and/or probe(s) (5) of the desired sequence is/are released from the at least one transport helper molecule part (3) by means of chemical, physical or enzymatic cleavage of the cleavable molecule part (4).

9. Method according to either claim 7 or claim 8,
**characterised in that**
the primer(s) and/or probe(s) (5) of the desired sequence is/are released from the at least one transport helper molecule part (3) by means of thermal cleavage of the cleavable molecule part (4).

10. Method according to claim 7,
**characterised in that**
the cleavable molecule part (4) is thermally cleaved during a denaturing step in the PCR reaction, preferably at 94 to 96°C.

11. Kit for feeding a primer (5) and/or a probe (5) into a closed PCR system (6),
**characterised in that**
the kit comprises a modified oligonucleotide (2) according to any of claims 1-6 and at least one auxiliary for releasing the primer (5) and/or the probe (5) from said modified oligonucleotide (2).

## Revendications

1. Utilisation d'un oligonucléotide modifié (2), comportant au moins une sonde (5) et/ou une primaire de PCR (5) de séquence prédéterminée pour l'éclusage de ladite au moins une sonde (5) et/ou primaire de PCR dans un système PCR fermé,
**caractérisée par le fait qu'**
un oligonucléotide (2) est choisi, lequel comporte au moins la ou les sondes (5) et/ou la ou les primaires de PCR (5), qui sont reliées à au moins une partie de molécule auxiliaire de transport (3) par au moins une partie de molécule clivable (4), où, par la partie de molécule auxiliaire de transport seule ou en combinaison avec d'autres parties de l'oligonucléotide modifié, les propriétés de l'oligonucléotide modifié sont réglées de telle sorte qu'il est apte à être lié au moins temporairement sur leurs surfaces comme molécules d'ADN ou d'ARN à longue chaîne sur la base d'une affinité de liaison élevée vis-à-vis d'une phase solide et est apte à être élué à partir de la phase solide comme molécules d'ADN ou d'ARN à longue chaîne, où l'oligonucléotide modifié est lié au moins temporairement conjointement avec l'ADN ou l'ARN de sonde sur cette phase solide, pendant que d'autres substances présentes dans la sonde restent dans la phase aqueuse et sont éliminées dans une étape de purification conjointement avec le liquide
et
après cette purification, dans une étape d'élution, ont lieu la dissolution de l'oligonucléotide modifié conjointement avec l'ADN ou l'ARN de sonde à partir de la phase solide et l'éclusage dans la région du système PCR fermé dans lequel doit avoir lieu la PCR et
par la suite la ou les primaires et la ou les sondes (5) de la séquence souhaitée est ou sont libérées par clivage de la partie de molécule clivable (4) à partir de ladite au moins une partie de molécule auxiliaire de transport (3).

2. Utilisation selon la revendication 1,
**caractérisée par le fait que**
la partie de molécule auxiliaire de transport (5) est un oligo- ou polynucléotide, de préférence un gène ou une partie d'un gène, dont la séquence est connue.

3. Utilisation selon la revendication 2,
**caractérisée par le fait que**
la partie de molécule auxiliaire de transport (3) ne s'hybride pas avec la séquence de la primaire de PCR (5) et/ou de la sonde (5) dans des conditions de PCR.

4. Utilisation selon l'une des revendications précédentes,
**caractérisée par le fait que**
la partie de molécule clivable (4) est clivable chimiquement, physiquement ou enzymatiquement.

5. Utilisation selon l'une des revendications précédentes,
**caractérisée par le fait que**
l'oligonucléotide modifié (2) présente une pluralité de parties de molécule qui sont choisies dans le groupe qui comporte des sondes (5) et des primaires de PCR (5).

6. Utilisation selon l'une des revendications précédentes,
**caractérisée par le fait qu'**
au moins une sonde (5) est liée par un emplacement de couplage avec une partie de molécule clivable (4), qui ne peut être allongée par des polymérases après le clivage de la partie de molécule clivable (4) dans des conditions de PCR.

7. Procédé d'éclusage d'une primaire et/ou d'une sonde dans un système PCR fermé (6)
**caractérisé par le fait qu'**
un oligonucléotide modifié (2) est choisi, lequel comporte au moins une sonde (5) et/ou une primaire (5) d'une séquence souhaitée ainsi qu'au moins une partie de molécule clivable (4) et au moins une partie de molécule auxiliaire de transport (3), où cette partie de molécule auxiliaire de transport se comporte dans son affinité à une phase solide comme un ADN ou ARN à amplifier de la sonde pour lier sur cette phase solide l'oligonucléotide modifié au moins temporairement conjointement avec l'ADN ou l'ARN de sonde, pendant qu'autres substances présentes dans la sonde restent dans la phase aqueuse et sont éliminées dans une étape de purification conjointement avec le liquide et
après cette purification, dans une étape d'élution, ont lieu la dissolution de l'oligonucléotide modifié conjointement avec l'ADN ou l'ARN de sonde à partir de la phase solide et l'éclusage dans la région du système PCR fermé dans lequel doit avoir lieu la PCR, et
par la suite la ou les primaires et/ou la ou les sondes (5) de la séquence souhaitée est ou sont libérées par clivage de la partie de molécule clivable (4) à partir de ladite au moins une partie de molécule auxiliaire de transport (3).

8. Procédé selon la revendication 7,
**caractérisé par le fait que**
la ou les primaires et/ou la ou les sondes (5) de la séquence souhaitée est ou sont libérées de ladite au moins une partie de molécule auxiliaire de transport (3) par clivage chimique, physique ou enzymatique de la partie de molécule clivable (4).

9. Procédé selon l'une des revendications 7 ou 8
**caractérisé par le fait que**
la ou les primaires et/ou la ou les sondes (5) de la séquence souhaitée est ou sont libérées de ladite au moins une partie de molécule auxiliaire de transport (3) par clivage thermique de la partie de molécule clivable (4).

10. Procédé selon la revendication 7,
**caractérisé par le fait que**
le clivage thermique de la partie de molécule clivable (4) a lieu pendant une étape de dénaturation de la réaction de PCR, de préférence à 94-96°C.

11. Kit pour l'éclusage d'une primaire (5) et/ou d'une sonde (5) dans un système PCR fermé (6)
**caractérisé par le fait que**
le kit comporte un oligonucléotide modifié (2) selon l'une des revendications 1- 6 ainsi qu'au moins un auxiliaire pour la libération de la primaire (5) et/ou de la sonde (5) à partir de cet oligonucléotide modifié.
